# EUROPEAN PATENT APPLICATION

(11) **EP 4 356 835 A1**
(43) Date of publication of application: **24.04.2024**
(21) Application number: 22202494.5
(22) Date of filing: 19.10.2022
(51) Int. Cl.: A61B 5/305, A61B 5/308, A61B 5/31, A61B 5/318, A61B 5/00

(54) **A LEAD SET FOR A BIO-POTENTIAL RECODING SYSTEM**

(71) Applicant: Koninklijke Philips N.V., 5656 AG Eindhoven (NL)
(72) Inventor: FRANCK, Christoph Florian, 5656AG Eindhoven (NL)
(74) Representative: Philips Intellectual Property & Standards

(57) **Abstract**

A lead set system is for delivering signals to a bio-potential recording unit. A lead set has a plurality of leads, each defining an input channel to the bio-potential recording unit. Correction information is stored as part of the lead set system (i.e. independent of the bio-potential recording unit) relating to the plurality of leads for use in interpreting the input channels, thereby to improve channel symmetry.

## Description

### FIELD OF THE INVENTION

This invention relates to a lead set for a bio-potential recording system. In particular it relates to the reduction of signal channel asymmetry in the signals collected by a lead set.

### BACKGROUND OF THE INVENTION

Bio-potential measurements such as electrocardiography (ECG/EKG) and electroencephalography (EEG) are an established part of clinical practice and furthermore used in other areas like research. Commercial recording systems usually comprise a recording unit that is connected to an electrode lead set. The electrode lead set in turn is connected to electrodes (adhesive, needle, etc.) placed on defined locations of the patient's body.

The recording system measures the small voltage differences that occur between the electrodes due to physiological electrical activity. The differential measurement is susceptible to interference from common-mode signals, as common-mode to differential-mode conversion (CM2DM) occurs if the behavior of the input channels is not identical before the difference operation. Differences in the behavior of the input channel is referred to as channel asymmetry.

US 11/147517 discloses a method for reducing the asymmetries in the electrical behavior of a recording device's input channel. The aim is in particular to reduce asymmetries that occur due to electronic component tolerances in the recording unit.

However, channel asymmetry in a bio-potential recording system may also be caused by the electrode lead sets. These are usually replaced more frequently than the recording device itself. They are considered supplies or accessories to the recording system. They may be single-use disposable accessories or re-useable.

Electrode lead sets can be classified as "passive" or "active". Passive lead sets only contain electrical conductors and passive electronic components like resistors, capacitors, inductors and diodes. Active lead sets additionally contain electronic components that amplify, buffer or digitally sample the signal such as operational amplifiers or analog-to-digital converters (ADC). Active lead sets are used to achieve an improved signal-to-noise ratio compared to passive lead sets by placing the amplifier, buffer or ADC as close to the patient as possible and limiting the length of the electrical connection traversed by the un-buffered signal.

The benefits of active lead sets are for example discussed in Dabbaghian A, Kassiri H, "An Active Electrode IC with Embedded Analog CMRR Enhancement for Interference- and Gain-Mismatch-Resilient EEG Recording". 2021 IEEE Biomedical Circuits and Systems Conference (BioCAS); DOI: 10.1109/BIOCAS49922.2021.9644952.

Regardless of whether the lead set is active or passive, if the lead set contains electronic components subject to tolerances, it will contribute to the input channel asymmetry. This degrades the common-mode rejection ratio (CMRR) of the recording setup and makes the recording more susceptible to noise and artefacts. Passive lead sets are usually, but not always, designed so that their contribution to the input channel asymmetries is negligible. Active lead sets, on the other hand, contain a significant part of the analog signal processing chain and therefore have a substantial influence on the input channel asymmetries. This is one reason that limits the benefits and applicability of active lead sets in practice.

Thus, there remains a problem that electrode lead sets, especially active electrode lead sets, contribute to the input channel asymmetries of a bio-potential recording system, and this degrades the quality and usefulness of the signal. It also limits the benefits of placing the signal processing elements of the recording system close to the patient in the case of active electrode lead sets.

### SUMMARY OF THE INVENTION

The invention is defined by the independent claims. Dependent claims define advantageous embodiments.

According to examples in accordance with an aspect of the invention, there is provided a lead set system for delivering signals to a bio-potential recording unit, comprising:
a lead set, comprising a plurality of leads, each defining an input channel to a bio-potential recording unit; and
stored fixed correction information relating to the plurality of leads for use in processing the input channels, thereby to improve channel symmetry.

The invention thus provides an electrode lead set with associated stored correction information. In combination, this is termed a "lead set system" in this text. The correction information can be accessed by the bio-potential recording unit to which the lead set is connected. The access to the correction information can use a wired or wireless means of communication.

The correction information is independent of the bio-potential recording unit, in that it forms part of the lead set system, which itself is for delivering signals to the bio-potential recording unit. In other words, the correction information is associated with the lead set alone and may be used by different bio-potential recording units.

The correction information is for example generated during manufacturing of the lead set, for example by applying a calibration signal to the input side of the lead set and recording the resulting signals at the output side of the lead set. The correction information is "fixed" in the sense that it is associated with the physical lead set and does not depend on the use, or intended use, of the lead set. Thus, the correction information is constant for each manufactured lead set.

By storing fixed correction information for a particular lead set, there is no need for a separate calibration or testing process as part of the use of the lead set. Instead, channel symmetry is improved in use based on the already-available correction information, which is provided with the lead set and independently of the bio-potential recording unit. No (further) performance measurements in respect of the lead set are required by the bio-potential recording unit. Indeed, no performance measurements of the lead set are conducted by the bio-potential recording unit because they are generated independently and in advance of use of the lead set.

The lead set is a detachable and sometimes even disposable part, and the lead sets themselves may have different electrical properties, either due to manufacturing or component tolerances, or even by design. The electrical behavior of an individual lead set may differ significantly from any simulation used during the calibration measurements of the recording device.

The matching/symmetry of the input channel is thus improved by taking calibration measurements of each produced lead set, and correction information is then stored.

The recorded output signals during the calibration testing can be processed into correction information in the form of a set of digital filter coefficients that the bio-potential recording unit applies to the input channel signals. Thus, sets of digital filters for equalization are calculated and stored in the lead set system.

In conventional manner, each lead is for example terminated by a contact electrode for placing against the skin.

The correction information for example specifies, or is used to derive, filter coefficients which are used as part of a correction procedure by applying a digital filter. These filter coefficients are for example filter coefficients for use after the analog to digital conversion.

The correction information may be encrypted. This ensures the correction information can only be used by authorized bio-potential recording units. The bio-potential recording unit contains the means (e.g., secret decryption key or a means to authenticate the recording unit to the lead set) to decrypt the correction information.

In one set of examples, the stored correction information may be remote from the lead set. In this case, the correction information is a separate entity to the lead set itself, but it is still remote from the bio-potential recording unit.

In another set of examples, the lead set comprises a digital storage device which stores the correction information. In this case, the correction information is stored in a device which is an integral part of the lead set.

The digital storage device preferably comprises a non-volatile memory. The correction information is thus static compensation data associated with the lead set.

The lead set system may comprise a connector for connecting the leads to a bio-potential recording unit, wherein the connector comprises the digital storage device. Thus, the storage is integrated into a connector of the lead set.

The lead set system may further comprise a trunk cable for connecting to the bio-potential recording unit, wherein the connector comprises a trunk cable connector between the plurality of leads and the trunk cable. In this arrangement, a digital storage device is part of a trunk cable connector.

The lead set system may further comprise:
a lead set connector to which the plurality of leads connect, and
a set of intermediate leads between the lead set connector and the trunk cable connector,
wherein the lead set connector comprises a further digital storage device.

In this arrangement, a digital storage device is part of a both a lead set connector and a trunk cable connector. This means different lead set segments may each have their own correction information.

The invention also provides a bio-potential recording system comprising:
the lead set system as defined above; and
a bio-potential recording unit,
wherein the bio-potential recording unit is configured to read the stored correction information to improve channel symmetry.

Channel symmetry is improved because the correction information enables the signal processing chain to compensate for transfer function differences between the plurality of leads.

This defines the combination of the lead set and the recording unit.

The bio-potential recording unit for example comprises digital filters, wherein the bio-potential recording unit is configured to use the correction information to configure the digital filters for each lead of the lead set. The digital filters are for example used after digital sampling of the analog to digital conversion.

The correction information may specify, or may be used to derive, filter coefficients which are used as part of a correction procedure.

The bio-potential recording unit or the lead set for example comprises an analogue to digital converter for each lead of the lead set, wherein the analogue to digital converters are configured to sample the signal of each lead in single-ended mode referenced against a reference.

This allows the calibration to be applied to each input channel signal before differences between individual input channels are calculated. The difference between input channels is derived by analog circuitry after the digital sampling operation.

The bio-potential recording unit may comprise a decryption system for decrypting encrypted correction information.

The invention also provides a method of processing signals received from a lead set, comprising:
receiving signals from lead of a lead set, each lead defining an input channel; and
accessing stored correction information relating to the leads of the lead set.
processing the input channels using the stored correction information, thereby to improve channel symmetry. The processing of the input channels for example comprises analog to digital conversion.

The invention also provides a computer program comprising computer program code which is adapted, when said program is run on a computer, to implement the method above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a system diagram of a bio-potential recording system.
Fig. 2 shows an example of a lead set between electrodes and a bio-potential recording unit with multiple sections.
Fig. 3 shows a first example of a lead set with local storage of correction information.
Fig. 4 shows a second example of a lead set with local storage of correction information.
Fig. 5 shows a third example of a lead set with remote storage of correction information.
Fig. 6 shows a method of processing signals received from a lead set.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a lead set system for delivering signals to a bio-potential recording unit. A lead set has a plurality of leads, each defining an input channel to the bio-potential recording unit. Calibration information is stored as part of the lead set system (i.e., independent of the bio-potential recording unit) relating to the plurality of leads for use in interpreting the input channels, thereby to improve channel symmetry.

The "channel symmetry" is improved when the signals reaching the bio-potential recording unit from the patient electrodes have undergone a similar signal transfer function. In particular, if the combined signal transfer function of a lead and an associated digital filter is matched for different leads, then the ability to extract the signals of interest at the bio-potential recording unit (after the digital filtering) is improved. In particular, common mode interference is more reliably eliminated, hence the common mode rejection ratio is improved. Thus, improving channel symmetry involves improving a matching of the signal transfer function of the input channels, i.e., the signal transfer function from the patient electrodes to a signal processing stage in the bio-potential recording unit. The signal processing stage involves comparing signals received from different channels.

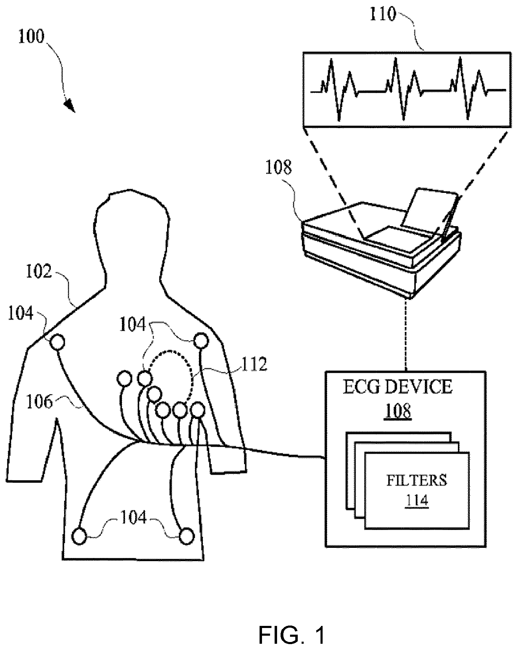

Fig. 1 shows a system diagram 100 of a bio-potential recording unit in the form of an ECG device 108 that uses one or more filters 114 to improve signal quality when performing electrophysiological measurements on a person 102. The filters 114 are specifically digital filters for channel equalization purposes.

The bio-potential recording unit 108 includes a lead set having leads 106 that provide a conductive pathway between electrodes 104 that are attached to the person 102 and the bio-potential recording unit 108. The electrodes 104 can provide electrical potentials across the body of the person 102, and the electrical potentials can be affected by the changes in polarity of the heart 112. The placement of the electrodes 104 is selected in order to capture the changes in polarity at different angles that correspond to vectors between the electrodes 104. As the electrical potentials of the electrodes 104 are modified by the changes in polarity of the heart 112, the bio-potential recording unit 108 can process the changes to the electrical potentials and generate a resulting signal 110 that represents the electrical activity associated with the heart 112.

However, because the changes in polarity of the heart 112 modify the electrical potentials of the electrodes 104 only slightly at times, differentiating the changes in electrical potential from electrical interference can prove difficult. Electrical interference can take the form of common mode interference, which can result from external sources, such as electrical line interference, or internal sources such as capacitive coupling between portions of the bio-potential recording unit 108.

The accuracy of the measurements thus depends on the quality of the signals being transmitted through the electrodes and their respective leads. In an ideal case, the calculation of voltage differences will mean that any signal components that affect all electrodes equally will be cancelled out. Thus, common mode interference is eliminated. However, if different leads have different transfer functions (hence distorting the signals reaching the bio-potential recording unit differently), or the amount of interference is too large compared to the signal, an overall pattern of electrical activity measured at the body can be rendered inaccurate for purposes of medical diagnoses.

Common mode interference can affect many types of electrophysiological measurements. In order to reduce and/or eliminate such interference, channels of the measurement system can be calibrated to identify filter parameters for individually mitigating common mode interference or voltage differences between groups of electrodes. Filters can be applied to electrode signals before voltage differences are calculated between electrodes.

In order to mitigate and/or eliminate the interference, the bio-potential recording unit 108 includes one or more hardware and/or software filters 114 for filtering the signals received at the electrodes 104. Each electrode 104 is assigned to a filter 114, and each coefficient for each electrode 104 can be provided to minimize time-domain differences and interference of signals from each electrode 104.

The system of Fig. 1 is known from US 11/147517.

In accordance with the teaching of US 11/147517, the coefficients for the filters 114 are derived during a calibration performed by the bio-potential recording unit 108, thus compensating in particular for the processing by the bio-potential recording unit 108.

The system of US 11/147517 takes into account the components of the input path that are present at the time of calibration. The input signals can either be applied directly to the inputs of the device under test, or a circuit that is an electrical equivalent of an average or worst-case lead set can be inserted before the inputs of the device.

The method in US 11/147517 does not consider the fact that individual lead sets may differ in their behavior. The calibration and correction based on this method will be most effective in practice if the recording device is used with a lead set whose properties are similar to the simulation circuit used during calibration. The more different the used lead set's behavior is from the simulation circuit, the less effective the correction is in practice.

This invention aims to address the blind spot of different lead set behavior.

The invention provides stored correction information as part of the lead set, so that the lead set and the correction information may together be considered to define a lead set system, and this lead set system is for delivering signals to a bio-potential recording unit (e.g. an ECG device). The correction information is for use in processing the input channels, thereby to improve channel symmetry, and it relates only to the electrical characteristics of the lead set alone.

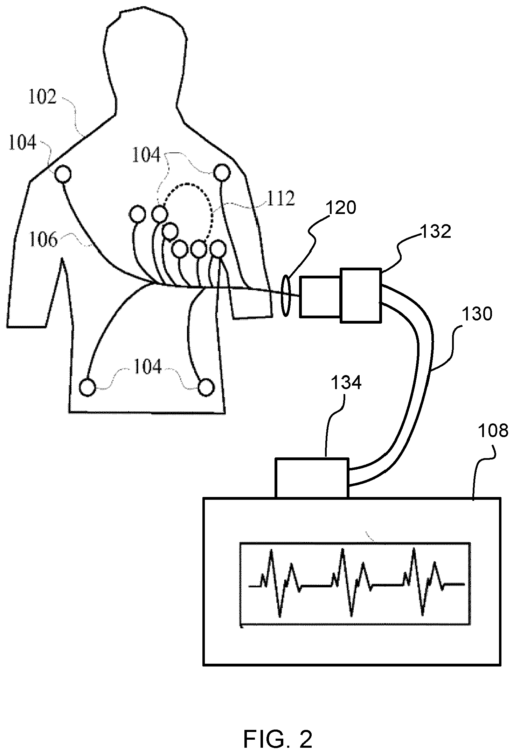

The leads 106 may connect directly to the bio-potential recording unit via a connector. However, Fig. 2 shows an example of a lead set between the electrodes 104 and the bio-potential recording unit 108 with multiple sections.

The leads 106 form a set of separate leads (as represented schematically at 120). The leads 106 of the set 120 connect to a trunk cable 130 for connecting to the bio-potential recording unit 108. A trunk cable connector 132 is between the set 120 of leads and the trunk cable 130. Another connector 134 is between the trunk cable 130 and the bio-potential recording unit 108.

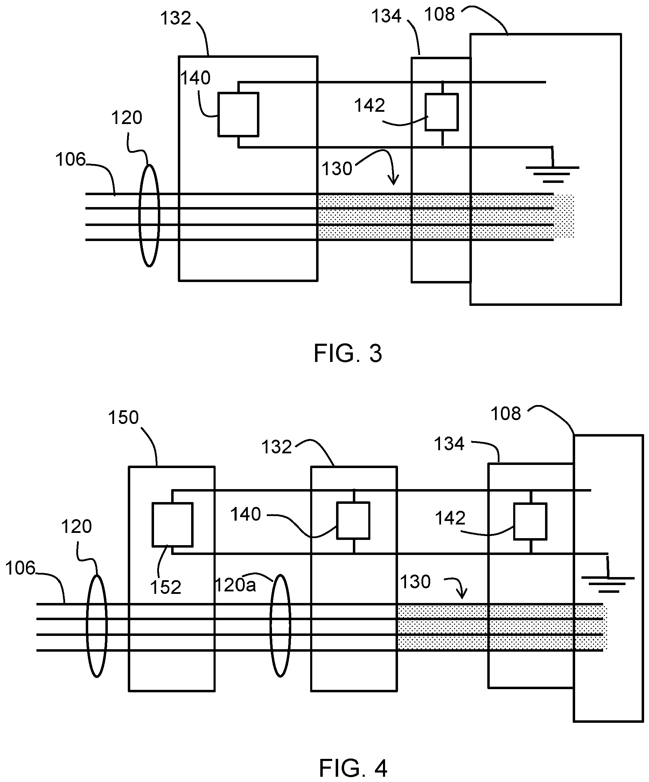

The invention makes use of stored correction information. In a first set of examples (Figures 3 and 4), the correction information is stored in one or more digital storage devices which form part of the lead set.

Fig. 3 shows a lead set comprising the set 120 of individual leads 106 which connect to a trunk connector 132, which then connects to a trunk cable 130. The trunk cable 130 connects to the ECG unit 108 via a further connector 134.

The trunk connector 132 has a digital storage device 140 and the further connector 134 has a digital storage device 142.

Each portion of the lead set thus has correction information relating to the characteristics of that portion of the lead set. This may be the portion between connectors at each end, or it may be the portion between the electrodes and a (most distal) connector.

Fig. 4 shows a lead set comprising the set 120 of individual leads 106 which connect to a lead set connector 150, which still connects to a set of individual leads 120a. There is then a trunk cable connector 132 which connects to a trunk cable 130 and a further connector 134 which connects to the bio-potential reading unit 108.

The three connectors each have data storage. The trunk connector 132 has storage device 140 and the further connector 134 has storage device 142. The lead set connector 150 has storage device 152.

The data stored in the three storage devices is accessible at the bio-potential recording unit 108 by wired connections which run alongside the leads.

In another example (not shown) the separate leads can connect directly to the ECG unit 108 via a single lead set connector. There is then only one storage device (for the characteristics of the leads from the electrodes to the lead set connector).

Each individual section has its own memory device for the storage of correction information specific to that section.

The ground connection to the memory devices is in the reading unit 108 as shown. In this example, the contents of the memory devices are retrieved using an interface requiring only a combined power and data connection and a ground connection. Other interfaces that use more connections are possible, as are interfaces that do not require electrical connections (e.g. NFC).

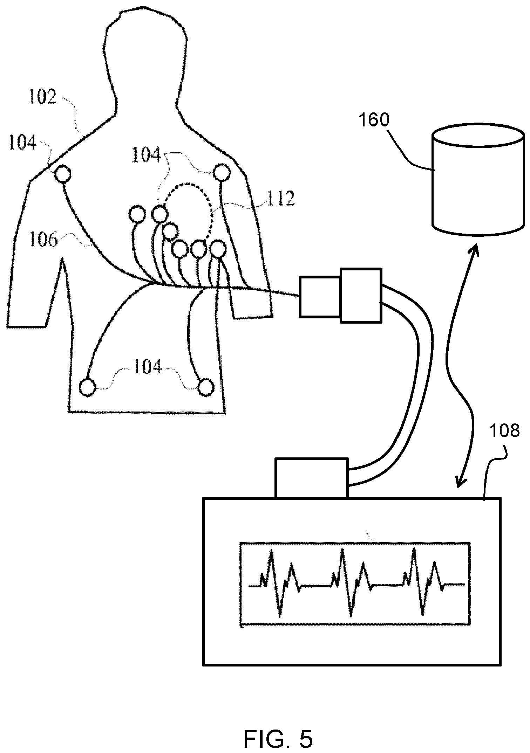

In a second set of examples illustrated by Fig. 5, the bio-potential recording unit 108 has access to the correction information from a remote memory 160. There is a wireless communication system between the bio-potential recording unit 108 and the remote memory 160.

The system of the invention extends the input channel calibration to the lead set and provides the lead set calibration as a separate function, before any use of the lead set. As lead sets are changed frequently, it is not desirable to store the correction information in the bio-potential recording unit (e.g., the ECG device 108) itself but instead to associate the correction information with the lead set itself.

By compensating for possible differences between the leads, the channel symmetry is improved as explained above, with the result that the common-mode rejection ratio is improved of the recording system. The measurement is less susceptible to noise and artefacts due to common-mode signals or interference. Measurements that are resilient to interference and artefacts are more useful for diagnostic and therapeutic decisions. This improves patient outcomes and the experiences of patients and staff.

According to the first set of examples, the correction information can be stored in the lead set, and the recording unit can then retrieve the information when a new lead set is connected. The digital storage device is preferably a non-volatile memory that is integrated in the lead set in the first set of examples. The non-volatile memory is used to store correction information specific to each individual lead set. The content of the digital storage device can be accessed by the bio-potential recording unit by a wired connection as explained above (UART, I2C, SPI, 1WIRE, etc.) but a wireless (NFC, etc.) connection may also be used.

The correction information allows the recording unit to improve the channel symmetry, i.e. to compensate for input channel asymmetry which may be caused by different transfer functions of the leads of the lead set. This correction information is generated during the production of the lead set and stored in the storage device. The correction information is generated for example by applying a calibration signal to the input side of the lead set and recording the resulting signal at the output side of the lead set. The behavior of each individual input channel is recorded and correction information in the form of channel-specific digital filter coefficients is derived from the recordings. The recorded output signal is thus processed into a set of digital filter coefficients that the bio-potential recording unit applies to the input channel signals.

The bio-potential recording unit (or the lead set, in case of lead sets with integrated analog-to-digital converters) samples the input channels in a single-ended mode, referenced against a stable internal reference. This allows the calibration to be applied to each input channel signal after the analog to digital sampling operation, before differences between individual input channels are calculated.

The storage device may contain means for storing the correction information in encrypted form. For example, the digital filter coefficients are stored in encrypted form to ensure they can only be used by authorized recording devices. The bio-potential recording unit contains the means (e.g., secret decryption key or a means to authenticate the recording unit to the lead set) to decrypt the correction information. This ensures that only authorized bio-potential recording units can make use of the correction information.

The storage device may also contain means for cryptographic authentication of the lead set by the controller. This allows the bio-potential recording unit to recognize authorized lead sets.

If the cable connecting the bio-potential recording unit and the electrodes on the patient consists of multiple individual segments connected in series (such as shown in Fig. 3), for example in the form of a trunk cable and attached lead sets, then any segment that contributes significantly to the input channel asymmetry has its own digital storage device containing the correction information specific to this segment. Segments closer to the bio-potential recording unit contain a means to allow communication with the digital storage devices of the more distant segment, regardless of whether the closer segment contains its own digital storage device. This may take the form of a hot-swappable bus like 1-WIRE, or by daisy-chaining communication interfaces that are not hot-swappable. The chain of communication may use several communication interfaces, for example USB to communicate with the digital storage device in the trunk cable and using 1-WIRE for communication between digital logic in the trunk cable and the attached lead set. The communication between the trunk cable and the attached lead set is relayed to the bio-potential recording unit vie the USB connection between it and the trunk cable.

For the system with remote storage of the correction information (Fig. 5), each lead set carries a unique identifier which can be read digitally and which is associated with the corresponding correction information in the database. Upon connection of the lead set, the bio-potential recording unit retrieves the unique identifier and uses it to retrieve the set of digital filter coefficients associated with the lead set from the database over a network connection. This method requires the bio-potential recording unit to be connected to the network with the database server.

As mentioned above, the filter coefficients may be used as part of the digital sampling of the analog signals collected at the electrodes. The filter coefficients define digital filters for the inputs of the electrode channels. The resulting filters minimize time-domain differences between signals of each input channel and a desired response. Furthermore, as a result of applying the filters to each electrode channel, common mode interference signals can be more readily mitigated and/or canceled.

The digital filtering is performed by correction filters, to implement a correction function after A/D conversion/sampling. It does not necessarily have to be the first processing step after A/D conversion - other, fixed digital filters may be applied first.

If the input paths of the lead set were to contain plain time delays, then the optimization process would generate filter coefficients that implement time delays so that the overall delays of each channel after correction are as similar as possible.

In reality, the situation is more complex, as the (linear) behavior of each input channel is described by its transfer function. The transfer function, as an approximation, gives a phase shift (delay) and a gain for each frequency. Thus, different frequencies may be delayed by different durations and attenuated by different factors.

The basic form of the transfer function is given by the electrical circuit or a suitable model. The more complex the transfer function, the more filter coefficients are necessary for the correction filter to achieve sufficient correction.

The optimization-based approach can compensate for any type of linear behavior. Linear transfer functions may be used of any complexity. Most cables can however be modelled with fairly simple transfer functions.

The invention can be applied in bio-potential recording systems such as electrocardiographs, electroencephalographs, or multi-parameter patient monitors. The invention enables improved performance of patient monitors and other electrophysiological recording devices, when the improved lead set system is used.

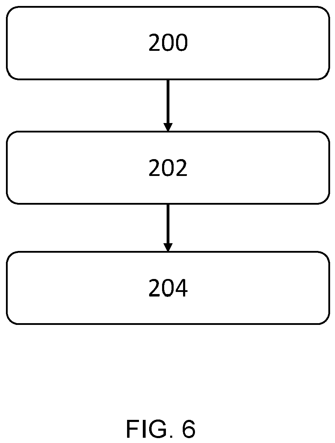

Fig. 6 shows a method of processing signals received from a lead set.

In step 200, signals are received from lead of a lead set, each lead defining an input channel.

In step 202, stored fixed correction information is accessed, relating to the leads of the lead set, such as digital filter coefficients as explained above, optionally encrypted.

In step 204 the input channels are processed using the stored correction information, thereby to improve channel symmetry.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A lead set system for delivering signals to a bio-potential recording unit (108), comprising:
a lead set (120), comprising a plurality of leads (106), each defining an input channel to the bio-potential recording unit, and
stored fixed correction information relating to the plurality of leads for use in processing the input channels, thereby to improve channel symmetry.

2. The lead set system of claim 1, wherein the correction information comprises digital filter coefficients.

3. The lead set of claim 1 or 2, wherein the correction information is encrypted.

4. The lead set system of any one of claims 1 to 3, wherein the stored correction information is remote from the lead set.

5. The lead set system of any one of claims 1 to 3, wherein the lead set comprises a digital storage device (140; 142; 152) which stores the correction information.

6. The lead set system of claim 5, wherein the digital storage device comprises a non-volatile memory.

7. The lead set system of claim 5 or 6, comprising a connector (134) for connecting the leads to a bio-potential recording unit, wherein the connector (134) comprises the digital storage device (142).

8. The lead set system of claim 7, further comprising a trunk cable (130) for connecting to the bio-potential recording unit (108), wherein the connector comprises a trunk cable connector (132) between the plurality (120) of leads of the lead set and the trunk cable (130).

9. The lead set system of claim 8, further comprising:
A lead set connector (150) to which the plurality of leads of the lead set connect, and
a set (120a) of intermediate leads between the lead set connector and the trunk cable connector (132),
wherein the lead set connector (150) comprises a further digital storage device (152).

10. A bio-potential recording system comprising:
the lead set system of any one of claims 1 to 9, and
a bio-potential recording unit (108),
wherein the bio-potential recording unit is configured to read the stored correction information to improve channel symmetry.

11. The system of claim 10, wherein the bio-potential recording unit comprises digital filters, wherein bio-potential recording unit is configured to use the correction information to configure the digital filters for each lead of the lead set.

12. The system of claim 10 or 11, wherein the bio-potential recording unit or the lead set comprises an analogue to digital converter for each lead of the lead set, wherein the analogue to digital converters are configured to sample the signal of each lead in single-ended mode referenced against a reference.

13. The system of any one of claims 10 to 12, wherein the bio-potential recording unit comprises a decryption system for decrypting encrypted correction information.

14. A method of processing signals received from a lead set, comprising:
(200) receiving signals from lead of a lead set, each lead defining an input channel, and
(202) accessing stored fixed correction information relating to the leads of the lead set,
(204) processing the input channels using the stored correction information, thereby to improve channel symmetry.

15. A computer program comprising computer program code which is adapted, when said program is run on a computer, to implement the method of claim 14.
